# EUROPEAN PATENT APPLICATION

(11) **EP 4 001 909 A1**
(43) Date of publication of application: **25.05.2022**
(21) Application number: 20841118.1
(22) Date of filing: 14.07.2020
(51) Int. Cl.: G01N 27/416, C02F 1/00, G01N 33/18, A01K 63/04

(54) **WATER QUALITY MEASURING SYSTEM**

(30) Priority: 16.07.2019 JP 2019131015
(71) Applicant: NGK SPARK PLUG CO., LTD., Mizuho-ku Nagoya-shi Aichi 467-8525 (JP)
(72) Inventor: TAKEHIRO Yoji, Nagoya-shi, Aichi 467-8525 (JP); TAMAI Kazusei, Nagoya-shi, Aichi 467-8525 (JP); PUTRA Gray Lawrence Sirosi, Nagoya-shi, Aichi 467-8525 (JP); TASHIMA Keisuke, Nagoya-shi, Aichi 467-8525 (JP); KAMEI Shunsuke, Nagoya-shi, Aichi 467-8525 (JP); KOJIMA Junji, Kizugawa-shi, Kyoto 619-0223 (JP); IWAMOTO Yasukazu, Kizugawa-shi, Kyoto 619-0223 (JP); NAGAI Hiroshi, Kizugawa-shi, Kyoto 619-0223 (JP)
(74) Representative: Grünecker Patent- und Rechtsanwälte PartG mbB
(86) International application number: PCT/JP2020/027386
(87) International publication number: WO 2021/010400

(57) **Abstract**

A water quality measuring system (100) includes a first introduction section (131) for introducing rearing water as a sampling target, and a first adding section (132) which adds an acid to the rearing water introduced by the first introduction section (131), and a nitrous acid sensor (28) whose measurement target is nitrous acid and which measures the measurement target concentration of the rearing water to which the acid has been added by the first adding section (132). The water quality measuring system (100) includes a second adding section (133) which adds a base to the rearing water introduced by the first introduction section (131), and an ammonia sensor (26) whose measurement target is ammonia and which measures the measurement target concentration of the rearing water to which the base has been added by the second adding section (133).

## Description

### TECHNICAL FIELD

The present invention relates to a water quality measuring system.

### BACKGROUND ART

A land-based aquaculture apparatus disclosed in Patent Literature 1 includes a culturing tank filled with culturing water (seawater) for culturing fishery products, an ammonia sensor for detecting the ammonia concentration of the water, and an ion sensor for detecting the concentration of a specific ion in the water. The ion sensor is, for example, a nitrate ion sensor. An ion electrode for detecting nitrate ion is connected to a reference electrode. An electromotive force between the two electrodes in a test solution is measured, and the nitrate ion concentration is calculated from the electromotive force.

### CITATION LIST

### PATENT LITERATURE

Patent Literature 1: Japanese Patent Application Laid-Open (kokai) No. 2003-52275

### SUMMARY OF INVENTION

### TECHNICAL PROBLEM

Incidentally, the ion electrode used in the ion sensor may detect not only a target ion to be detected but also ions of other species (interference ions) whose characteristics are similar to those of the target ion, thereby affecting the electromotive force. Accordingly, in the case where detection of the target ion is attempted by using the ion sensor for a solution which contains not only the target ion but also a large amount of interference ions, due to the influence of the interference ions, the target ion concentration cannot be detected accurately in some cases. For example, in the case where the ammonia and/or nitrous acid concentration of saline water for rearing an aquatic organism is detected by the ion sensor, the ammonia and/or nitrous acid concentration cannot be measured accurately due to the influence of salt contents; i.e., sodium ion, potassium ion, or chloride ion.

The present invention has been accomplished so as to solve at least part of the above-described problem and its object is to provide a water quality measuring system which can accurately measure the nitrous acid or ammonia concentration of rearing water which is used for rearing an aquatic organism and which contains salt contents.

### SOLUTION TO PROBLEM

A water quality measuring system which is one solution of the present invention is a water quality measuring system which measures properties of rearing water which is used for rearing an aquatic organism and which contains a salt content, comprising:
an introduction section which introduces the rearing water as a sampling target;
an adding section which adds an acid to the rearing water introduced by the introduction section; and
a diaphragm type ion sensor whose measurement target is nitrous acid and which measures the measurement target concentration of the rearing water to which the acid has been added by the adding section.

In the above-described water quality measuring system, an acid is added by the adding section to the rearing water introduced by the introduction section, whereby nitrite ion contained in the rearing water can be transformed to nitrous acid gas. The nitrous acid gas produced in this manner can be detected selectively by the diaphragm type ion sensor. As a result, it is possible to prevent adverse effect of ions still contained in the water on detection of nitrous acid, thereby enabling accurate measurement of the nitrous acid concentration.

In particular, this water quality measuring system is configured in such a manner that an acid is added by the adding section to the rearing water introduced by the introduction section, whereby the pH of the rearing water is adjusted. Since it is unnecessary to perform a preliminary treatment for pH adjustment for the entire rearing water (from which sample rearing water is obtained) before being introduced into the introduction section, an environment in which the aquatic organism is reared is unlikely to be adversely affected.

Also, this water quality measuring system suppresses the influence of disturbances (aeration, movement of organisms, etc.) more easily as compared with a configuration in which a direct-insertion-type sensor is used for the rearing water (from which sample rearing water is obtained) before being introduced into the introduction section.

A water quality measuring system which is another solution of the present invention is a water quality measuring system which measures properties of rearing water which is used for rearing an aquatic organism and which contains a salt content, comprising:
an introduction section which introduces the rearing water as a sampling target;
an adding section which adds a base to the rearing water introduced by the introduction section; and
a diaphragm type ion sensor whose measurement target is ammonia and which measures the measurement target concentration of the rearing water to which the base has been added by the adding section.

In the above-described water quality measuring system, a base is added by the adding section to the rearing water introduced by the introduction section, whereby ammonium ion contained in the rearing water can be transformed to ammonia gas. The ammonia gas produced in this manner can be detected selectively by the diaphragm type ion sensor. As a result, it is possible to prevent adverse effect of ions still contained in the water on detection of ammonia, thereby enabling accurate measurement of the ammonia concentration.

In particular, this water quality measuring system is configured in such a manner that a base is added by the adding section to the rearing water introduced by the introduction section, whereby the pH of the rearing water is adjusted. Since it is unnecessary to perform a preliminary treatment for pH adjustment for the entire rearing water (from which sample rearing water is obtained) before being introduced into the introduction section, an environment in which the aquatic organism is reared is unlikely to be adversely effected.

Also, this water quality measuring system suppresses the influence of disturbances (aeration, movement of organisms, etc.) more easily as compared with a configuration in which a direct-insertion-type sensor is used for the rearing water (from which sample rearing water is obtained) before being introduced into the introduction section.

The above-described water quality measuring system may further comprise a second introduction section which introduces the rearing water, which is the sampling target, through a passage different from the introduction section; an adjustment section which performs a reduction treatment on the rearing water introduced by the second introduction section; a color forming section which adds a color former to the rearing water chemically reduced by the adjustment section, the color former developing a color by reacting with nitrite ion; a color sensor which measures the color of the rearing water to which the color former has been added by the color forming section; and a calculation section which calculates the nitrate ion concentration of the rearing water, which is the sampling target, on the basis of the measurement target concentration detected by the diaphragm type ion sensor and the color of the rearing water detected by the color sensor.

In this water quality measuring system, the adjustment section performs reduction treatment on the rearing water introduced by the second introduction section, whereby nitrate ion contained in the rearing water can be converted to nitrite ion. Since the water quality measuring system is configured to cause the color forming section to add a color former, which reacts with nitrite ion and develops a color, to the rearing water chemically reduced in this manner, the chemically reduced rearing water develops a color corresponding to the nitrite ion concentration. Thus, the calculation section can calculate the nitrate ion concentration of the rearing water on the basis of the measurement target concentration (nitrous acid concentration) detected by the diaphragm type ion sensor and the color of the rearing water detected by the color sensor.

The above-described water quality measuring system may further comprise a filtration section for filtering the rearing water. The diaphragm type ion sensor may measure the measurement target concentration of the rearing water filtered by the filtration section.

In this water quality measuring system, since filtration of the rearing water is performed by the filtration section before measurement of the measurement target concentration, remaining feed, metabolites, turbid components, etc., which cause clogging of a flow passage and color variation, can be removed before the concentration measurement, whereby the concentrations of the measurement targets can be measured more accurately.

The above-described water quality measuring system may further comprise a measurement section which measures the concentration of a component which is contained in the rearing water before flowing through the adding section and which differs from the measurement target.

In this water quality measuring system, the concentration of a component different from the measurement target is measured by the measurement section before the pH of the rearing water is adjusted by the adding section. Therefore, the concentration of the component different from the measurement target can be measured without receiving the influence of pH adjustment and the influence of a component added for pH adjustment.

The above-described water quality measuring system may further comprise a measurement section which measures the concentration of a component which is contained in the rearing water before flowing through the adjustment section and which differs from the measurement target.

In this water quality measuring system, the concentration of a component different from the measurement target is measured by the measurement section before reduction treatment is performed on the rearing water by the adjustment section. Therefore, the concentration of the component different from the measurement target can be measured without receiving the influence of the reduction treatment.

### ADVANTAGEOUS EFFECTS OF INVENTION

The present invention enables accurate measurement of the nitrous acid or ammonia concentration of rearing water which is used for rearing an aquatic organism and contains salt contents.

### BRIEF DESCRIPTION OF DRAWINGS

[FIG. 1] FIG. 1 is an explanatory diagram used for briefly describing a production system in a first embodiment.
[FIG. 2] FIG. 2 is a block diagram exemplifying the electrical configuration of the production system of FIG. 1.
[FIG. 3] FIG. 3 is an explanatory diagram used for describing a main portion of a water quality measuring system.
[FIG. 4] FIG. 4 is an explanatory diagram used for describing a first sensor section.
[FIG. 5] FIG. 5 is an explanatory diagram used for describing a second sensor section.

### DESCRIPTION OF EMBODIMENTS

### <First embodiment

### 1. Outline of production system

A first embodiment will be described with reference to the drawings.

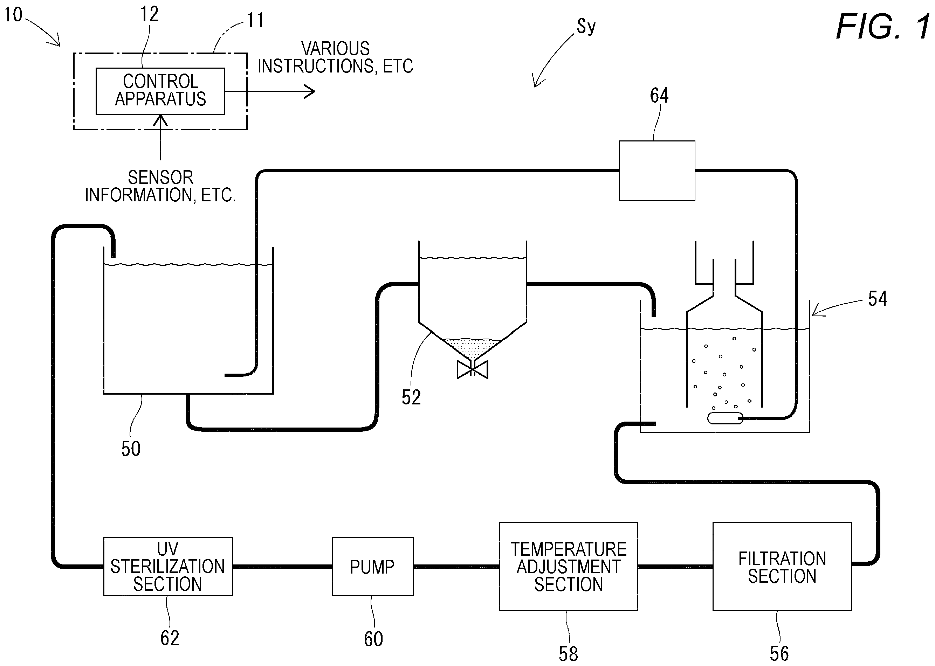

A production system Sy shown in FIG. 1 is a system for rearing an aquatic organism and is provided at a production base where the aquatic organism is reared. The following is description of an example in which the production system Sy is configured in the form of a closed-recirculating-type land-based aquaculture aquaculture system.

Notably, in the following description, a crustacean such as prawn is shown as an example of the aquatic organism, and an example in which the crustacean is cultured by the production system Sy will be described. However, the aquatic organism is not limited to this example and may be fishes such as sea bream, shellfishes such as scallop, or seaweeds such as *Wakame* seaweed.

The production system Sy shown in FIG. 1 includes a production base side apparatus 10, a rearing tank 50, a sedimentation tank 52, a bubble separation tank 54, filtration section 56, a temperature adjustment section 58, a pump 60, a UV sterilization section 62, an oxygen supply apparatus 64, etc. The rearing tank 50 is a water tank in which rearing water is stored and an aquatic product is reared. The sedimentation tank 52 is a tank in which sold substances contained in the rearing water are sedimented for sold liquid separation. The bubble separation tank 54 is a tank in which bubbles (air bubbles) are generated by a bubble separation apparatus and contaminants in the rearing water are caused to adhere to the bubbles for separation. The filtration section 56 is a facility for filtering the rearing water and includes, for example, a tank which enables successive performance of physical filtration and biological filtration. The temperature adjustment section 58 is a facility for adjusting the temperature of the rearing water and is configured, for example, in the form of a tank which can heat and cool the rearing water. The pump 60 is a pump for circulating the rearing water. The UV sterilization section 62 is a facility for sterilizing the flowing rearing water by using a UV germicidal lamp. The oxygen supply apparatus 64 is an apparatus for supplying oxygen into the rearing tank 50 so as to optimize the amount of oxygen dissolved in the rearing water.

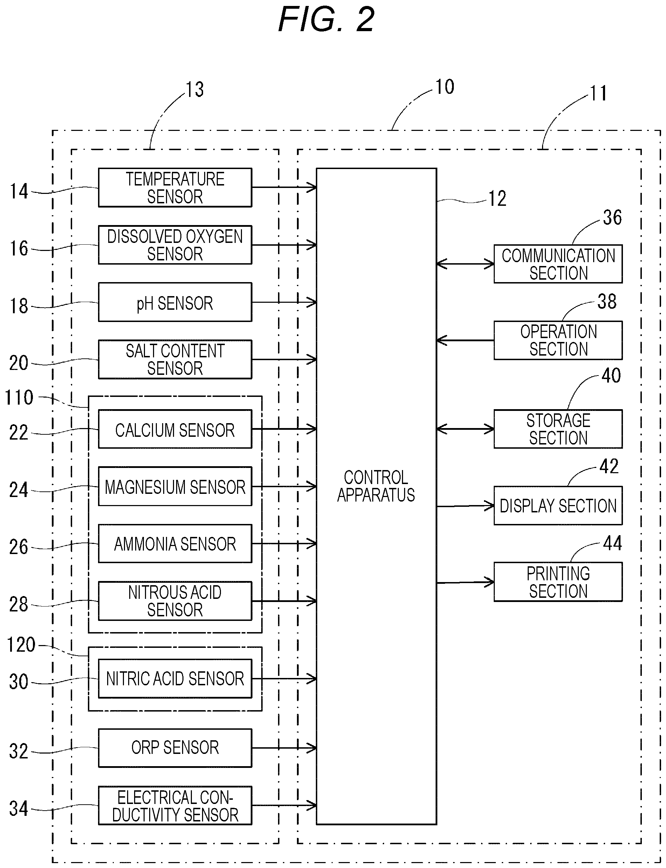

As shown in FIG. 2, the production base side apparatus 10 includes an information processing apparatus 11 and a sensor group 13 and is configured in such a manner that pieces of information can be transmitted to and received from other apparatuses via a communication network. The information processing apparatus 11 is configured, for example, in the form of a computer and mainly includes a control apparatus (calculation section) 12, a communication section 36, an operation section 38, a storage section 40, a display section 42, a printing section 44, etc. The control apparatus 12 is an information processing apparatus including a CPU and can perform various types of computation, control, and information processing. The communication section 36 is an apparatus which performs wired communication or wireless communication with external apparatuses in accordance with a well-known scheme. The operation section 38 is a well-known input device such as a keyboard, a mouse, a touch panel, or the like. The display section 42 is a well-known display apparatus such as a display. The printing section 44 is a well-known printing apparatus such as a printer.

The sensor group 13 includes a temperature sensor 14, a dissolved oxygen sensor 16, a pH sensor 18, a salt content sensor 20, a calcium sensor 22, a magnesium sensor 24, ammonia sensor 26, a nitrous acid sensor 28, a nitric acid sensor 30, a redox potential sensor 32 (hereinafter also referred to as the ORP sensor 32), an electrical conductivity sensor 34, etc. The temperature sensor 14 has a configuration for enabling detection of temperature at one or more locations in the system Sy and detects, for example, the temperature of the rearing water within the rearing tank 50. The dissolved oxygen sensor 16 detects the dissolved oxygen concentration of the rearing water within the rearing tank 50. The pH sensor 18 detects the pH of the rearing water within the rearing tank 50. The salt content sensor 20 detects the salinity concentration of the rearing water within the rearing tank 50. The calcium sensor 22 detects the calcium concentration of the rearing water within the rearing tank 50. The magnesium sensor 24 detects the magnesium concentration of the rearing water within the rearing tank 50. The ammonia sensor 26 detects the ammonia concentration of the rearing water within the rearing tank 50. The nitrous acid sensor 28 detects the nitrous acid concentration of the rearing water within the rearing tank 50. The nitric acid sensor 30 detects the nitric acid concentration of the rearing water within the rearing tank 50. The ORP sensor 32 detects the redox potential of the rearing water within the rearing tank 50. The electrical conductivity sensor 34 detects the electrical conductivity of the rearing water within the rearing tank 50. Notably, each sensor may include one detecting portion or a plurality of detecting portions for detecting a detection target.

### 2. Configuration of water quality measuring system

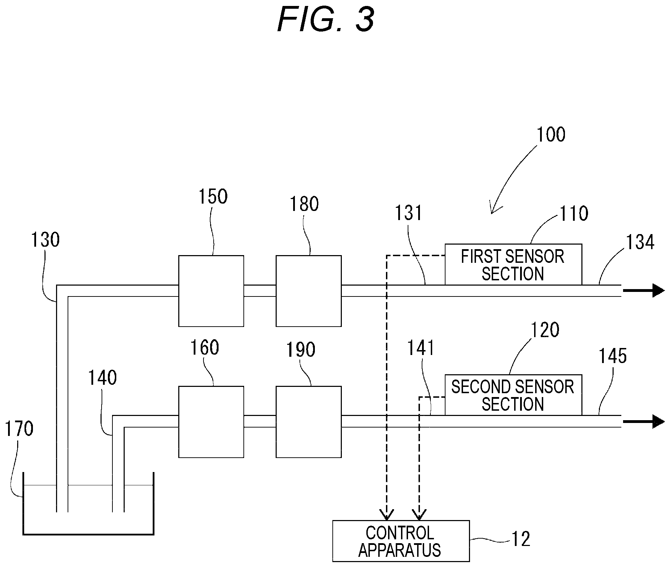

The water quality measuring system 100 of the first embodiment shown in FIG. 3 is a system for measuring the properties of the rearing water stored in the rearing tank 50. The rearing water is water which is used for rearing an aquatic organism and which contains salt contents (for example, seawater, brackish water, artificial seawater, or the like). The water quality measuring system 100 measures the concentrations of measurement targets (ammonia gas, nitrous acid gas, ammonium ion, nitrite ion, nitrate ion, etc.) contained in the rearing water.

As shown in FIG. 3, the water quality measuring system 10 includes a first sensor section 110, a second sensor section 120, the control apparatus 12, a first flow line 130, a second flow line 140, a first pump 150, a second pump 160, a first filtration section 180, a second filtration section 190, etc. The water quality measuring system 100 sucks the rearing water within a sampling container 170 by using the first pump 150 and the second pump 160 so that the rearing water flows through the first flow line 130 and the second flow line 140. In the water quality measuring system 100, the rearing water flowing through the first flow line 130 and the rearing water flowing through the second flow line 140 are introduced into the first sensor section 110 and the second sensor section 120, respectively, so as to measure the concentrations of the measurement targets contained in the rearing water. The rearing water, which is a sampling target, is sampled from the rearing tank 50 and is stored in the sampling container 170.

The first pump 150 is a well-known pump and is provided in the first flow line 130. The first pump 150 pumps the rearing water from the sampling container 170 to the first flow line 130 and feeds the pumped rearing water through the first filtration section 180 to a first introduction section (introduction section) 131 communicating with the first sensor section 110. The first filtration section 180 removes foreign substances from the rearing water by filtration. The first introduction section 131 is a passage for introducing the rearing water (sampling target) into the first sensor section 110. The second pump 160 is a well-known pump and is provided in the second flow line 140. The second pump 160 pumps the rearing water from the sampling container 170 to the second flow line 140 and feeds the pumped rearing water through the second filtration section 190 to a second introduction section (introduction section) 141 communicating with the second sensor section 120. The second filtration section 190 removes foreign substances from the rearing water by filtration. The second introduction section 141 is a passage for introducing the rearing water (sampling target) into the second sensor section 120. Upstream portions of the first flow line 130 and the second flow line 140 may be replaced with a common line, and the flow of the rearing water may be divided into two lines by using a valve or the like before flowing into the first sensor section 110 and the second sensor section 120.

### 3. Description of first sensor section

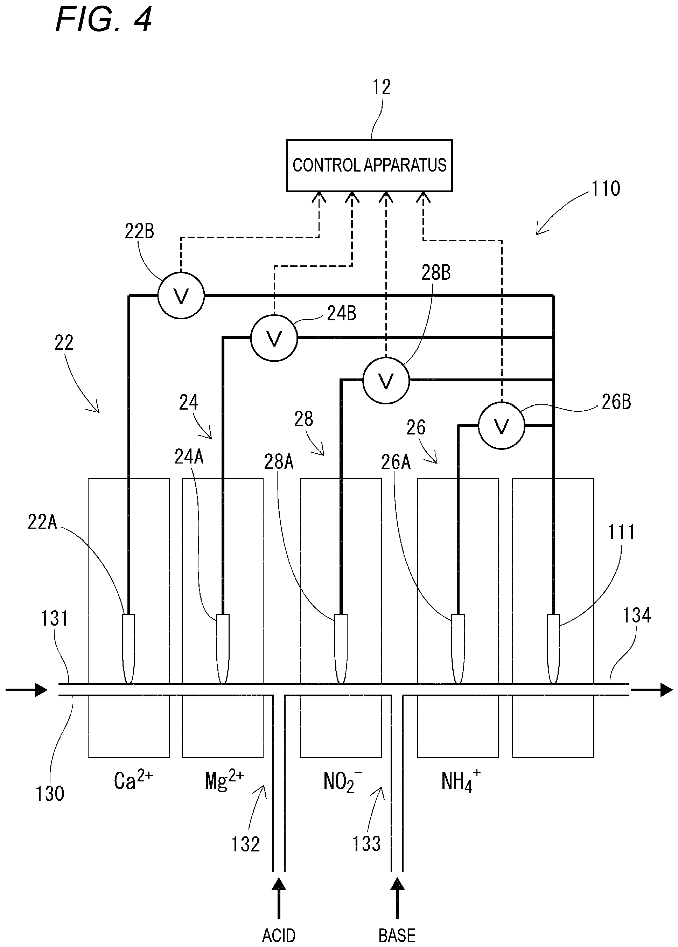

The first sensor section 110 shown in FIG. 4 measures the concentrations of the measurement targets (ammonia gas, nitrous acid gas, ammonium ion, nitrite ion) contained in the rearing water flowing through the first flow line 130. As shown in FIG. 4, the first sensor section 110 includes the calcium sensor (measurement section) 22, the magnesium sensor (measurement section) 24, the nitrous acid sensor 28, the ammonia sensor 26, and a reference electrode 111.

As shown in FIG. 4, the calcium sensor 22 is provided in the first flow line 130 to be located downstream of and adjacent to the first introduction section 131. The calcium sensor 22 includes an ion electrode 22A and a first potentiometer 22B. The ion electrode 22A is configured, for example, in the form of a well-known liquid membrane ion electrode. The ion electrode 22A has an electrode film which selectively reacts with calcium ion and generates an electromotive force in response to the ion concentration. The ion electrode 22A is immersed into the rearing water flowing through the first flow line 130 on the downstream side of the first introduction section 131. The ion electrode 22A generates an electrode potential in response to the calcium ion concentration of the rearing water. The first potentiometer 22B is electrically connected to the ion electrode 22A and the reference electrode 111. The reference electrode 111 is an electrode which is configured in the form of a well-known reference electrode (comparison electrode) and generates a standard potential which serves as a reference for the electrode potential of the ion electrode 22A. The reference electrode 111 is provided in the first flow line 130 to be located downstream of and adjacent to the ammonia sensor 26, which will be described later. The first potentiometer 22B measures the difference between the electrode potential of the ion electrode 22A and the electrode potential of the reference electrode 111 and outputs a measurement result (a signal indicating a potential difference in response to the calcium ion concentration of the rearing water) to the control apparatus 12.

As shown in FIG. 4, the magnesium sensor 24 is provided in the first flow line 130 to be located downstream of and adjacent to the calcium sensor 22 (specifically, the ion electrode 22A). The magnesium sensor 24 includes an ion electrode 24A and a second potentiometer 24B. The ion electrode 24A is configured, for example, in the form of a liquid membrane ion electrode. The ion electrode 24A is immersed into the rearing water flowing through the first flow line 130 on the downstream side of the ion electrode 22A. The ion electrode 24A generates an electrode potential in response to the magnesium ion concentration of the rearing water having passed through the calcium sensor 22. The second potentiometer 24B is electrically connected to the ion electrode 24A and the reference electrode 111. The second potentiometer 24B measures the difference between the electrode potential of the ion electrode 24A and the electrode potential of the reference electrode 111 and outputs a measurement result (a signal indicating a potential difference in response to the magnesium ion concentration of the rearing water) to the control apparatus 12.

The nitrous acid sensor 28 measures the nitrous acid (measurement target) concentration of the rearing water after addition of an acid to the rearing water by a first adding section 132. As shown in FIG. 4, the nitrous acid sensor 28 is provided in the first flow line 130 to be located downstream of and adjacent to the magnesium sensor 24, which is provided downstream of and adjacent to the magnesium sensor 24. The first adding section 132 is a passage for adding an acid (for example, sulfuric acid) to the rearing water introduced by the first introduction section 131. As a result of addition of an acid by the first adding section 132, nitrite ion contained in the rearing water can be transformed into nitrous acid gas. For example, the pH of the rearing water is adjusted to 1.5 or lower by adding an acid to the rearing water by the first adding section 132.

The nitrous acid sensor 28 is configured in the form of a well-known diaphragm type ion sensor and includes an ion electrode 28A and a third potentiometer 28B. The ion electrode 28A is configured in the form of a well-known diaphragm type ion electrode. The ion electrode 28A is configured, for example, such that nitrous acid gas passing through a diaphragm is dissolved into an electrolytic solution within the electrode, and an electromotive force in response to the pH of the internal solution which changes due to nitrous acid gas is generated. In this manner, the ion electrode 28A can detect nitrous acid gas selectively. As a result, it is possible to accurately measure the nitrous acid concentration by preventing adverse effects of ions still contained in the water (interference ions) on detection of nitrous acid. The ion electrode 28A is immersed into the rearing water flowing through the first flow line 130 on the downstream side of the first adding section 132. The ion electrode 28A generates an electrode potential in response to the concentration of nitrous acid gas contained in the rearing water having passed through the first adding section 132 (namely, nitrous acid gas which is composed of nitrous acid gas contained in the rearing water from the beginning before passing through the first adding section 132 and nitrous acid gas transformed from nitrite ion as a result of passage through the first adding section 132). The third potentiometer 28B is electrically connected to the ion electrode 28A and the reference electrode 111. The third potentiometer 28B measures the difference between the electrode potential of the ion electrode 28A and the electrode potential of the reference electrode 111 and outputs a measurement result (a signal indicating the potential difference in response to the concentration of the gas composed of nitrous acid gas contained in the rearing water from the beginning and nitrous acid gas transformed from nitrite ion) to the control apparatus 12.

The ammonia sensor 26 measures the ammonia (measurement target) concentration of the rearing water after addition of a base by a second adding section 133. As shown in FIG. 4, the ammonia sensor 26 is provided in the first flow line 130 to be located downstream of and adjacent to the second adding section 133, which is provided downstream of and adjacent to the nitrous acid sensor 28. The second adding section 133 is a passage for adding a base (for example, sodium hydroxide aqueous solution) to the rearing water introduced by the first introduction section 131. As a result of addition of a base by the second adding section 133, ammonium ion contained in the rearing water can be transformed into ammonia gas. For example, the pH of the rearing water is adjusted to 11.5 or higher by adding a base to the rearing water at the second adding section 133.

The ammonia sensor 26 is configured in the form of a well-known diaphragm type ion sensor and includes an ion electrode 26A and a fourth potentiometer 26B. The ion electrode 26A is configured in the form of a well-known diaphragm type ion electrode. The ion electrode 26A is configured, for example, such that ammonia gas passing through a diaphragm is dissolved into an electrolytic solution within the electrode, and an electromotive force in response to the pH of the internal solution which changes due to ammonia acid gas is generated. In this manner, the ion electrode 26A can detect ammonia gas selectively. As a result, it is possible to accurately measure the ammonia concentration by preventing adverse effect of ions still contained in the water (interference ions) on detection of ammonia. The ion electrode 26A is immersed into the rearing water flowing through the first flow line 130 on the downstream side of the second adding section 133. The ion electrode 26A generates an electrode potential in response to the concentration of ammonia gas contained in the rearing water having passed through the second adding section 133 (namely, ammonia gas which is composed of ammonia gas contained in the rearing water from the beginning before passing through the second adding section 133 and ammonia gas transformed from ammonium ion as a result of passage through the second adding section 133). The fourth potentiometer 26B is electrically connected to the ion electrode 26A and the reference electrode 111. The fourth potentiometer 26B measures the difference between the electrode potential of the ion electrode 26A and the electrode potential of the reference electrode 111 and outputs a measurement result (a signal indicating the potential difference in response to the concentration of the gas composed of ammonia gas contained in the rearing water from the beginning and ammonia gas transformed from ammonium ion) to the control apparatus 12. The rearing water having passed through the ammonia sensor 26 is discharged from a first discharge section 134 after passing through the reference electrode 111.

The control apparatus 12 calculates the calcium ion concentration on the basis of the measurement result (signal indicating the potential difference in response to the calcium ion concentration of the rearing water) output from the calcium sensor 22 and stores the calcium ion concentration in the storage section 40. Similarly, the control apparatus 12 calculates the magnesium ion concentration on the basis of the measurement result (signal indicating the potential difference in response to the magnesium ion concentration of the rearing water) output from the magnesium sensor 24 and store the magnesium ion concentration in the storage section 40. Also, the control apparatus 12 calculates the nitrous acid gas concentration on the basis of the measurement result (signal indicating the potential difference in response to the concentration of the gas composed of nitrous acid gas contained in the rearing water from the beginning and nitrous acid gas transformed from nitrite ion) output from the nitrous acid sensor 28 and stores the nitrous acid concentration in the storage section 40. Similarly, the control apparatus 12 calculates the ammonia gas concentration on the basis of the measurement result (signal indicating the potential difference in response to the concentration of the gas composed of ammonia gas contained in the rearing water from the beginning and ammonia gas transformed from ammonium ion) output from the ammonia sensor 26 and stores the ammonia gas concentration in the storage section 40. The control apparatus 12 transmits pieces of information regarding these concentrations stored in the storage section 40 to other apparatuses through the communication section 36, displays the pieces of information on the display section 42, and prints the pieces of information by using the printing section 44.

### 4. Description of second sensor section

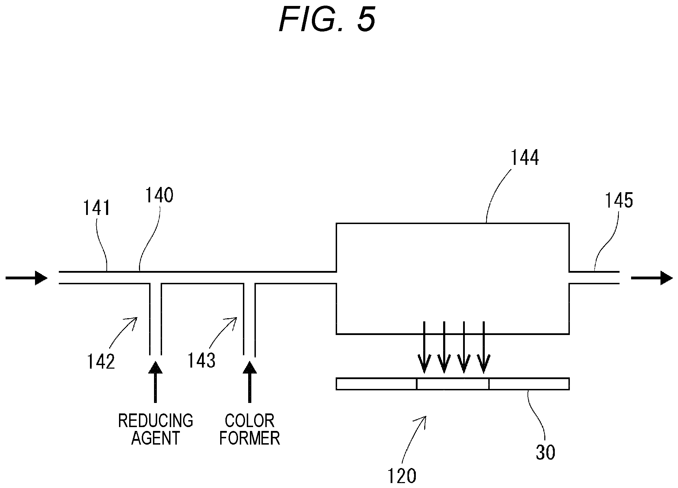

The second sensor section 120 shown in FIG. 5 measures the measurement target (nitrite ion contained in the rearing water from the beginning and nitrite ion transformed from nitrate ion) concentration of the rearing water flowing through the second flow line 140. As shown in FIG. 5, the second sensor section 120 is provided in the second flow line 140. The second sensor section 120 includes the nitric acid sensor 30. The nitric acid sensor 30 is configured in the form of a well-known color sensor. The nitric acid sensor 30 obtains color information (information which specifies a color) of liquid (measurement target). The second flow line 140 introduces the rearing water to be sampled into the second sensor section 120 through a second introduction section 141, which is a passage different from the first introduction section 131.

An adjustment section 142 is provided in the second flow line 140 to be located downstream of and adjacent to the second introduction section 141. The adjustment section 142 is a passage for adding a reducing agent (for example, zinc) to the rearing water introduced by the second introduction section 141. As a result of addition of a reducing agent by the adjustment section 142, nitrate ion contained in the rearing water can be transformed into nitrite ion.

In the second flow line 140, a color forming section 143 is provided downstream of and adjacent to the adjustment section 142. The color forming section 143 is a passage for adding a color former (for example, naphthylethylenediamine) to the rearing water containing the reducing agent added by the adjustment section 142. The rearing water develops a color when the color former reacts with nitrite ion. The color former causes the rearing water to develop a color in response to the nitrite ion concentration of the rearing water.

In the second flow line 140, a measurement area 144 is provided downstream of and adjacent to the color forming section 143. The measurement area 144 is an area where the rearing water having passed through the color forming section 143 is temporarily stored. The nitric acid sensor 30 is disposed in the vicinity of the measurement area 144 so that the rearing water becomes the measurement target of the nitric acid sensor 30. The nitric acid sensor 30 obtains the color information (information which specifies a color) of the rearing water. The nitric acid sensor 30 outputs the obtained color information to the control apparatus 12. The rearing water having passed through the measurement area 144 is discharged from a second discharge section 145.

The control apparatus 12 calculates the concentration of nitrite ion (nitrite ion contained in the rearing water from the beginning and nitrite ion transformed from nitrate ion) on the basis of the color information output from the nitric acid sensor 30 (information representing a color in response to the nitrite ion concentration of the rearing water) and stores the calculated nitrite ion concentration in the storage section 40. For example, pieces of information representing colors which are produced by nitrite ion-containing water as a result of addition of the color former (for example, each piece of information is obtained by quantifying the light and shadow of color) and pieces of information representing different nitrite ion concentrations are stored in the storage section 40 beforehand in such a manner that the pieces of information representing colors are related to the pieces of information representing different nitrite ion concentrations. The control apparatus 12 calculates the nitrite ion concentration from the color information output from the nitric acid sensor 30 on the basis of the correspondence relationship stored in the storage section 40.

The control apparatus 12 calculates the nitrate ion concentration of the rearing water (sampling target) on the basis of the measurement target concentration (nitrous acid gas concentration and nitrite ion concentration) detected by the first sensor section 110 and the color of the rearing water detected by the nitric acid sensor 30. Specifically, the control apparatus 12 calculates the nitrate ion concentration of the rearing water (sampling target) on the basis of the nitrous acid gas concentration detected by the first sensor section 110 (the concentration of a gas composed of nitrous acid gas contained in the rearing water from the beginning and nitrous acid gas transformed from nitrite ion) and the nitrite ion concentration calculated from the color information output from the nitric acid sensor 30 (the concentration of nitrite ion, including nitrite ion contained in the rearing water from the beginning and nitrite ion transformed from nitrate ion). More specifically, the control apparatus 12 determines the nitrate ion concentration of the rearing water (sampling target) by subtracting the nitrous acid gas concentration detected by the first sensor section 110 (the concentration of a gas composed of nitrous acid gas contained in the rearing water from the beginning and nitrous acid gas transformed from nitrite ion) from the nitrite ion concentration calculated from the color information output from the nitric acid sensor 30 (the concentration of nitrite ion, including nitrite ion contained in the rearing water from the beginning and nitrite ion transformed from nitrate ion). The control apparatus 12 stores the calculated nitrate ion concentration in the storage section 40. The control apparatus 12 transmits a piece of information regarding the calculated nitrate ion concentration to other apparatuses through the communication section 36, displays the pieces of information on the display section 42, and prints the pieces of information by using the printing section 44.

### 5. Effects

In the above-described water quality measuring system 100, an acid is added by the first adding section 132 to the rearing water introduced by the first introduction section 131, whereby nitrite ion contained in the rearing water can be transformed to nitrous acid gas. The nitrous acid gas produced in this manner can be detected selectively by the nitrous acid sensor 28. As a result, it is possible to prevent adverse effect of ions still contained in the water on detection of nitrous acid, thereby enabling accurate measurement of the nitrous acid concentration.

In particular, the water quality measuring system 100 is configured in such a manner that an acid is added by the first adding section 132 to the rearing water introduced by the first introduction section 131, whereby the pH of the rearing water is adjusted. Since it is unnecessary to perform a preliminary treatment for pH adjustment for the entire rearing water (from which sample rearing water is obtained) before being introduced into the first introduction section 131, an environment in which the aquatic organism is reared is unlikely to be adversely effected.

Also, this water quality measuring system 100 suppresses the influence of disturbances (aeration, movement of organisms, etc.) more easily as compared with a configuration in which a direct-insertion-type sensor is used for the rearing water (from which sample rearing water is obtained) before being introduced into the first introduction section 131.

In the above-described water quality measuring system 100, a base is added by the second adding section 133 to the rearing water introduced by the first introduction section 131, whereby ammonium ion contained in the rearing water can be transformed to ammonia gas. The ammonia gas produced in this manner can be detected selectively by the ammonia sensor 26. As a result, it is possible to prevent adverse effect of ions still contained in the water on detection of ammonia, thereby enabling accurate measurement of the ammonia concentration.

In particular, the water quality measuring system 100 is configured in such a manner that an acid is added by the second adding section 133 to the rearing water introduced by the first introduction section 131, whereby the pH of the rearing water is adjusted. Since it is unnecessary to perform a preliminary treatment for pH adjustment for the entire rearing water (from which sample rearing water is obtained) before being introduced into the first introduction section 131, the environment in which the aquatic organism is reared is unlikely to be adversely effected.

Also, this water quality measuring system 100 suppresses the influence of disturbances (aeration, movement of organisms, etc.) more easily as compared with a configuration in which a direct-insertion-type sensor is used for the rearing water (from which sample rearing water is obtained) before being introduced into the first introduction section 131.

The above-described water quality measuring system 100 includes the second introduction section 141 for introducing the rearing water (sampling target) through a passage different from the first introduction section 131; the adjustment section 142 for adding a reducing agent to the rearing water introduced by the second introduction section 141; the color forming section 143 for adding a color former, which reacts with nitrite ion and develops a color, to the rearing water containing the reducing agent added by the adjustment section 142; the nitric acid sensor 30 for measuring the color of the rearing water containing the color former added by the color forming section 143; and the control apparatus 12 for calculating the nitrate ion concentration of the rearing water (sampling target) on the basis of the measurement target concentration detected by the nitrous acid sensor 28 and the color of the rearing water detected by the nitric acid sensor 30.

This water quality measuring system 100 can convert nitrate ion contained in the rearing water to nitrite ion by causing the adjustment section 142 to add a reducing agent to the rearing water introduced by the second introduction section 141. Since the water quality measuring system 100 is configured to cause the color forming section 143 to add a color former, which reacts with nitrite ion and develops a color, to the rearing water whose pH has been adjusted in this manner, the pH adjusted rearing water develops a color corresponding to the nitrite ion concentration. Thus, the control apparatus 12 can calculate the nitrate ion concentration of the rearing water on the basis of the measurement target concentration (nitrous acid concentration) detected by the nitrous acid sensor 28 and the color of the rearing water detected by the nitric acid sensor 30.

The above-described water quality measuring system 100 includes the first filtration section 180 and the second filtration section 190 for filtering the rearing water. The first sensor section 110 measures the measurement target concentration of the rearing water filtered by the first filtration section 180, and the second sensor section 120 measures the measurement target concentration of the rearing water filtered by the second filtration section 190.

In this water quality measuring system 100, since filtration of the rearing water is performed by the first filtration section 180 and the second filtration section 190 before measurement of the measurement target concentration, interference ions, etc. can be removed before measurement of the concentrations, whereby the concentrations of the measurement targets can be measured more accurately.

In the above-described water quality measuring system 100, the calcium sensor 22 measures the concentration of a component (calcium ion) which is contained in the rearing water before passing through the first adding section 132 and the second adding section 133 and which differs from the measurement target. The magnesium sensor 24 measures the concentration of a component (magnesium ion) which is contained in the rearing water before passing through the first adding section 132 and the second adding section 133 and which differs from the measurement target.

In this water quality measuring system 100, the concentrations of components (calcium ion and magnesium ion) different from the measurement target are measured by the calcium sensor 22 and the magnesium sensor 24 before the pH of the rearing water is adjusted by the first adding section 132 and the second adding section 133. Therefore, the concentrations of components (calcium ion and magnesium ion) different from the measurement target can be measured without receiving the influence of pH adjustment and the influence of a component added for pH adjustment.

### <Other embodiments>

The present invention is not limited to the embodiment explained by the above description and the drawings, and, for example, the following examples fall within the technical scope of the present invention.

In the description of the first embodiment, the control apparatus 12 calculates the nitrate ion concentration by subtracting the nitrous acid gas concentration detected by the first sensor section 110 (the concentration of a gas composed of nitrous acid gas contained in the rearing water from the beginning and nitrous acid gas transformed from nitrite ion) from the nitrite ion concentration calculated from the color information output from the nitric acid sensor 30. However, the control apparatus 12 may calculate the nitrate ion concentration by subtracting only the concentration of nitrous acid gas transformed from nitrite ion from the nitrite ion concentration calculated from the color information output from the nitric acid sensor 30. For example, in the first sensor section 110, the concentration of nitrous acid gas contained in the rearing water from the beginning is measured by the nitrous acid sensor 28 without addition of an acid to the rearing water from the first introduction section 131. Subsequently, the concentration of nitrous acid gas transformed from nitrite ion can be determined by subtracting the concentration of nitrous acid gas contained in the rearing water from the beginning, which is measured without addition of an acid, from the nitrous acid gas concentration detected by the first sensor section 110 described in the first embodiment (the concentration of a gas composed of nitrous acid gas contained in the rearing water from the beginning and nitrous acid gas transformed from nitrite ion).

In the description of the first embodiment, only the ammonia gas concentration and the nitrous acid gas concentration are measured. However, through measurement of the pH and temperature of the rearing water, not only the ammonia gas concentration and the nitrous acid gas concentration but also the ammonium ion concentration and the nitrous acid ion concentration can be calculated from the measured ammonia gas concentration and nitrous acid gas concentration, the pH, the temperature, and the ionization constants of ammonia and nitrous acid, which are well known.

In the description of the first embodiment, prawn is shown as an example of the aquatic organism cultured in the production system Sy. However, the aquatic organism may be an organism (other than prawn) of *Decapoda*, *Malacostraca*, *Crustacea,* or *Arthropoda.*

In the description of the first embodiment, an example in which the nitrite ion concentration is measured by the second sensor section 120 after addition of a reducing agent by the adjustment section 142 is shown. However, instead of adding a reducing agent, the reduction treatment may be performed by a well-known method such as electrolytic reduction.

Also, in the description of the first embodiment, the calcium ion concentration and the magnesium ion concentration are measured in the first sensor section 110 before the rearing water flows through the first adding section 132. However, the present invention is not limited to such a configuration. For example, calcium ion and magnesium ion may be measured in the second sensor section 120 before the rearing water flows through the adjustment section 142.

In the description of the first embodiment, the control apparatus 12, the communication section 36, the operation section 38, the storage section 40, the display section 42, the printing section 44 are incorporated into the single information processing apparatus 11. However, the present invention is not limited to such a configuration. Some sections of the information processing apparatus 11 may be separated and data and instructions may be exchanged between the information processing apparatus 11 and the separated sections by wired or wireless communication.

### REFERENCE SIGNS LIST

- 12:: control apparatus (calculation section)
- 22:: calcium sensor (measurement section)
- 24:: magnesium sensor (measurement section)
- 26:: ammonia sensor (diaphragm type ion sensor)
- 28:: nitrous acid sensor (diaphragm type ion sensor)
- 30:: nitric acid sensor (color sensor)
- 100:: water quality measuring system
- 110:: first sensor section
- 120:: second sensor section
- 131:: first introduction section (introduction section)
- 132:: first adding section
- 133:: second adding section
- 141:: second introduction section
- 142:: adjustment section
- 143:: color forming section
- 180:: first filtration section
- 190:: second filtration section
- Sy:: production system

## Claims

1. A water quality measuring system which measures properties of rearing water which is used for rearing an aquatic organism and which contains a salt content, comprising:
an introduction section which introduces the rearing water as a sampling target;
an adding section which adds an acid to the rearing water introduced by the introduction section; and
a diaphragm type ion sensor whose measurement target is nitrous acid and which measures the measurement target concentration of the rearing water to which the acid has been added by the adding section.

2. A water quality measuring system which measures properties of rearing water which is used for rearing an aquatic organism and which contains a salt content, comprising:
an introduction section which introduces the rearing water as a sampling target;
an adding section which adds a base to the rearing water introduced by the introduction section; and
a diaphragm type ion sensor whose measurement target is ammonia and which measures the measurement target concentration of the rearing water to which the base has been added by the adding section.

3. A water quality measuring system according to claim 1, further comprising:
a second introduction section which introduces the rearing water, which is the sampling target, through a passage different from the introduction section;
an adjustment section which performs a reduction treatment on the rearing water introduced by the second introduction section;
a color forming section which adds a color former to the rearing water chemically reduced by the adjustment section, the color former developing a color by reacting with nitrite ion;
a color sensor which measures the color of the rearing water to which the color former has been added by the color forming section; and
a calculation section which calculates the nitrate ion concentration of the rearing water, which is the sampling target, on the basis of the measurement target concentration detected by the diaphragm type ion sensor and the color of the rearing water detected by the color sensor.

4. A water quality measuring system according to any one of claims 1 to 3, further comprising a filtration section for filtering the rearing water,
wherein the diaphragm type ion sensor measures the measurement target concentration of the rearing water filtered by the filtration section.

5. A water quality measuring system according to any one of claims 1 to 4, further comprising a measurement section which measures the concentration of a component which is contained in the rearing water before flowing through the adding section and which differs from the measurement target.

6. A water quality measuring system according to claim 3 or according to claim 4 or 5 depending from claim 3, further comprising a measurement section which measures the concentration of a component which is contained in the rearing water before flowing through the adjustment section and which differs from the measurement target.
